# EUROPÄISCHE PATENTSCHRIFT

(11) **EP 3 612 314 B1**
(45) Veröffentlichungstag und Bekanntmachung des Hinweises auf die Patenterteilung: **07.06.2023**
(21) Anmeldenummer: 18719153.1
(22) Anmeldetag: 19.04.2018
(51) Int. Cl.: B05B 5/00, B05B 5/035, B05B 5/043, B05B 5/16, B05B 12/08, B05B 5/053, B05B 9/08, B05B 11/00, A61M 35/00, A45D 34/00, A45D 34/02, B05B 1/14, B05B 9/04

(54) **VERFAHREN ZUR STEUERUNG EINES ELEKTROSTATISCHEN ZERSTÄUBERS FÜR FLÜSSIGKEITEN**
METHOD FOR CONTROLLING AN ELECTROSTATIC ATOMISER FOR LIQUIDS
PROCÉDÉ POUR COMMANDER UN PULVÉRISATEUR ÉLECTROSTATIQUE DESTINÉ À PULVÉRISER DES LIQUIDES

(30) Priorität: 21.04.2017 DE 102017108614
(43) Veröffentlichungstag der Anmeldung: 26.02.2020
(73) Patentinhaber: J. Wagner GmbH, 88677 Markdorf (DE)
(72) Erfinder: BARTHELMES, Jan, 88682 Salem (DE); GÖHRING, Alfred, 88682 Salem (DE); JELTSCH, Thomas, 88048 Friedrichshafen (DE); STOHL, Holger, 88677 Markdorf (DE); BISCHOFBERGER, Urban, 9442 Berneck (CH)
(74) Vertreter: Otten, Roth, Dobler & Partner mbB Patentanwälte
(86) Internationale Anmeldenummer: PCT/EP2018/060121
(87) Internationale Veröffentlichungsnummer: WO 2018/193069

(56) Entgegenhaltungen:
- EP-A1- 1 504 823
- EP-A1- 3 613 403
- DE-A1- 1 522 540
- JP-A- S5 817 864
- JP-A- 2013 094 719
- US-A1- 2009 184 186
- US-A1- 2011 040 147
- US-A1- 2012 207 651

## Beschreibung

Die Erfindung betrifft ein Verfahren zur Steuerung eines elektrostatischen Zerstäubers für Flüssigkeiten gemäß dem Oberbegriff des Anspruchs 1.

Elektrostatische Zerstäubung umfasst im Sinne der vorliegenden Erfindung alle Zerstäubungsvorgänge, welche Flüssigkeiten mit Effekten der Einwirkung einer Hochspannung zerstäuben. Insbesondere auch elektrohydrodynamische Effekte sowie elektrokinetische Effekte sind von dem Begriff dieser Art von Zerstäubung umfasst. Im Sinne der Erfindung kann unter einer elektrostatischen Zerstäubung auch eine elektrohydrodynamische Zerstäubung aufgefasst werden.

Aus der US 2010/0116897 A1 ist ein Verfahren zur Steuerung eines elektrostatischen Zerstäubers für Flüssigkeiten bekannt, wobei der Zerstäuber einen Flüssigkeitstank und eine Fördervorrichtung für Flüssigkeit aus dem Flüssigkeitstank, eine Hochspannungsquelle sowie mindestens eine Zerstäuberdüse für die Zerstäubung von Flüssigkeit umfasst und wobei die mindestens eine Zerstäuberdüse an der Hochspannungsquelle angeschlossen ist.

Aus der EP 1 504 823 A1 ist eine Spritzbeschichtungseinrichtung bekannt, welche ein Steuergerät enthält, durch welches elektrische Energie über eine elektrische Leitung einer elektrischen Spritzvorrichtung zuführbar ist, welche zum Sprühen von Beschichtungsmaterial auf einen zu beschichtenden Gegenstand ausgebildet ist und mindestens eine Hochspannungselektrode zum elektrostatischen Aufladen des Beschichtungsmaterials mittels einer Hochspannung aufweist; wobei das Steuergerät zum Einstellen von mindestens einem Beschichtungsbetriebs-Parameter ausgebildet ist; und welche mindestens ein optisches Anzeigegerät zum optischen Anzeigen von mindestens einer Beschichtungsbetriebs-Information enthält, wobei das Anzeigegerät ein extern vom Steuergerät positionierbares Anzeigegerät ist, dass das Steuergerät einen Sender zur leitungslosen Datenübertragung der mindestens einen anzuzeigenden Beschichtungsbetriebs-Information und das externe Anzeigegerät einen Empfänger zum leitungslosen Datenempfang der mindestens einen Beschichtungsbetriebs-Information ausgebildet ist.

Unter dem Anschluss der Zerstäuberdüse an der Hochspannungsquelle ist jedwede Wechselwirkung zu verstehen, welche die elektrostatische, insbesondere die elektrohydrodynamische Zerstäubung durch die Hochspannung ermöglicht. Eine Zerstäuberdüse kann dabei aus leitfähigem Material, aber auch aus nicht leitfähigem Material bestehen, um die notwendigen Effekte herbei zu führen.

Ein weiterer elektrostatischer Zerstäuber zum Auftragen eines Films ist aus der EP 3366270 A1 bekannt.

Der Erfindung liegt die Aufgabe zugrunde, ein Verfahren zur Steuerung eines elektrostatischen Zerstäubers für Flüssigkeiten vorzuschlagen, durch welches ein Sprühvorgang überwachbar und verbesserbar ist.

Diese Aufgabe wird ausgehend von den Merkmalen des Oberbegriffs des Anspruchs 1 durch die kennzeichnenden Merkmale des Anspruchs 1 gelöst. In den jeweiligen Unteransprüchen sind vorteilhafte und zweckmäßige Weiterbildungen angegeben.

Bei dem Verfahren zur Steuerung eines elektrostatischen Zerstäubers für Flüssigkeiten ist es vorgesehen, dass mittels Sensormitteln einer Kontrollelektronik an wenigstens einer der Zerstäuberdüsen die Spannung und/oder die Stromstärke erfasst werden und/oder dass mittels Sensormitteln der Kontrollelektronik die Spannung und/oder die Stromstärke an der Hochspannungsquelle erfasst werden. Hierdurch ist es möglich, Rückschlüsse auf die Arbeitsweise und das Arbeitsergebnis des Zerstäubers zu ziehen und mittels der Kontrollelektronik zu Verbesserung des Sprühergebnisses oder zur Vermeidung von Fehlern lenkend einzugreifen.

Bezüglich der Vorteile der weiteren Ansprüche wird auf die Beschreibung der Figuren verwiesen.

Im Sinne der Erfindung kann unter einer elektrostatischen Zerstäubung auch eine elektrohydrodynamische Zerstäubung aufgefasst werden.

Im Sinne der Erfindung kann ein Gitter auch als eine Anordnung mehrerer nebeneinander angeordneter Spalten sowie untereinander angeordneter Zeilen aufgefasst werden. Auch eine Dreieck-Symmetrie ist als Gitteranordnung denkbar.

Im Sinne der Erfindung ist unter einer Flüssigkeit jedwede für das Auftragen durch elektrostatische Zerstäubung geeignete Flüssigkeit aufzufassen. Im Sinne der Erfindung ist insbesondere vorgesehen, dass die Flüssigkeit ein Kosmetikum, z.B. ein Sonnenschutzmittel, eine Lotion oder ein Deodorant ist. Die Flüssigkeit kann auch eine flüssige Farbe oder Lack oder dergleichen sein. Auch andere Flüssigkeiten, z.B. ein Insektenschutzmittel, sind denkbar.

Weitere Einzelheiten der Erfindung werden in der Zeichnung anhand von schematisch dargestellten Ausführungsbeispielen beschrieben.

Hierbei zeigt:
- Figur 1:: eine Schnittansicht durch eine schematische Darstellung einer ersten Ausführungsvariante eines Zerstäubers, wobei die Zerstäuberdüsen in einer Ruhestellung stehen;
- Figur 2:: eine Schnittansicht durch die Darstellung der Figur 1, wobei die Zerstäuberdüsen in einer Arbeitsstellung stehen und
- Figur 3:: eine Seitenansicht auf die Figur 1 im Bereich der Zerstäuberdüsen.

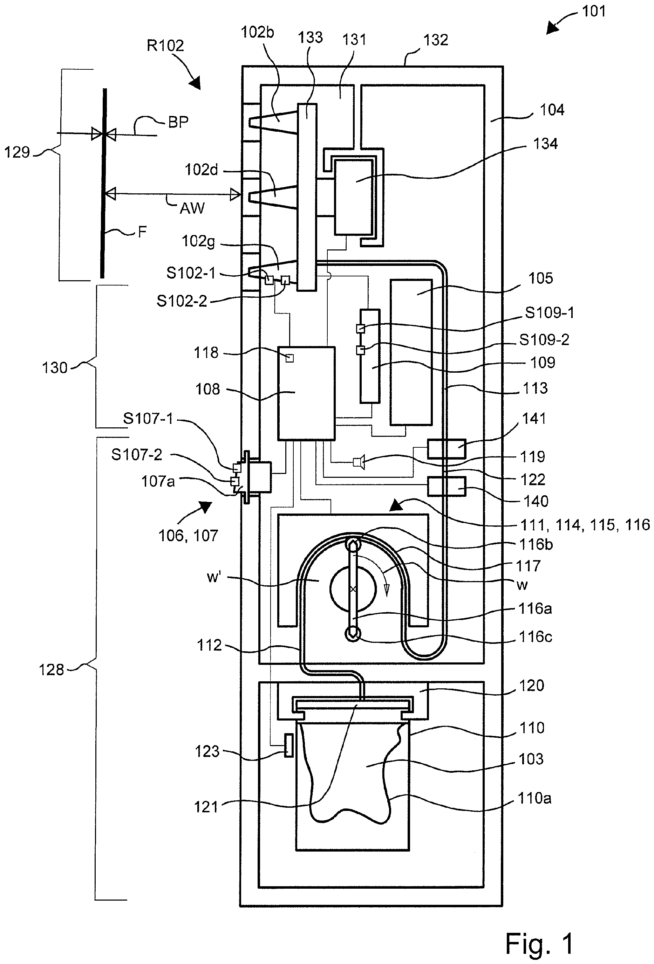

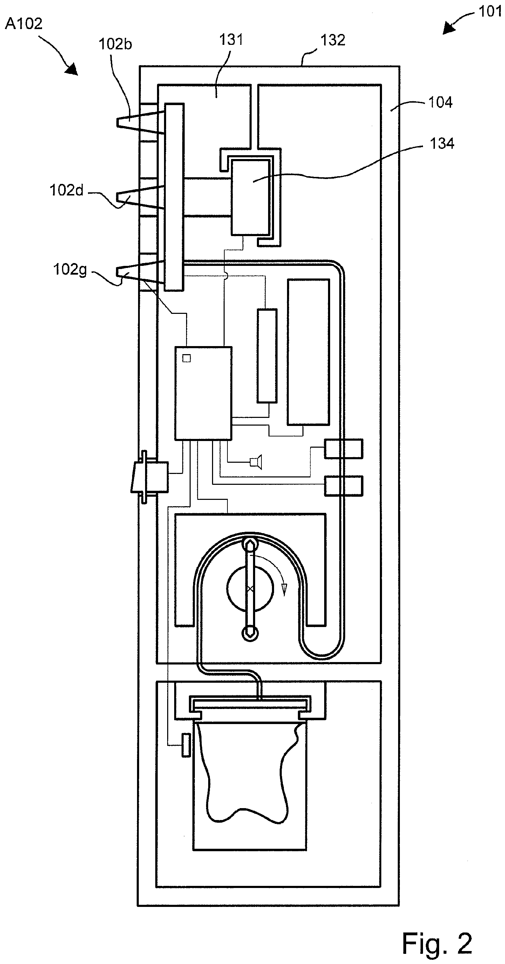

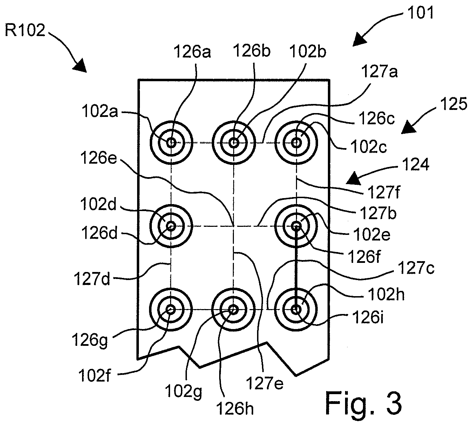

In der Figur 1 ist eine Schnittansicht durch eine schematische Darstellung eines Zerstäubers 101 gezeigt, wobei Zerstäuberdüsen 102a bis 102h in einer Ruhestellung R102 stehen. In der Figur 2 ist der in der Figur 1 gezeigte Zerstäuber 101 mit Zerstäuberdüsen 102a bis 102h gezeigt, welche in einer Arbeitsstellung A102 stehen. Die Figur 3 zeigt schließlich eine Seitenansicht auf den in der Figur 1 dargestellten Zerstäuber 101 im Bereich seiner Zerstäuberdüsen 102a bis 102h.

Die dargetellte Fassung weißt eine Vielzahl von Zerstäuberdüsen auf. Es ist jedoch ebenfalls erfindungsgemäß und zweckmäßig, eine geringere Anzahl an Zerstäuberdüsen, die im Rahmen des Anspruchs 1 liegt, insbesondere zwischen zwei und fünf Zerstäuberdüsen vorzusehen, je nachdem, welche Anforderungen die zu zerstäubende Flüssigkeit an die Geometrie und Anordnung der Düsen im Zusammenhang mit den elektrostatischen Effekten stellt.

Der elektrostatische Zerstäuber 101 ist für eine elektrostatische Zerstäubung von Flüssigkeiten 103 vorgesehen. Hierbei werden unter Flüssigkeiten insbesondere Kosmetika, aber z.B. auch Farben und Lacke verstanden. Der Zerstäuber 101 umfasst ein Gehäuse 104, eine elektrische Energiequelle 105, ein Aktivierungsmittel 106, welches als elektrischer Taster 107 bzw. elektronischer Kontakt (Kapazitiv-, Reed-, Hall-Sensor) ausgeführt ist, eine Kontrollelektronik 108, eine Hochspannungsquelle 109, einen Flüssigkeitstank 110, eine Fördervorrichtung 111 und die erwähnten Zerstäuberdüsen 102a bis 102h. Die Fördervorrichtung 111 ist zwischen dem Flüssigkeitstank 110 und den Zerstäuberdüsen 102a bis 102h angeordnet. Hierbei ist die Fördervorrichtung 111 durch eine erste Leitung 112 mit dem Flüssigkeitstank 110 verbunden ist und hierbei ist die Fördervorrichtung 111 durch eine zweite Leitung 113 mit den Zerstäuberdüsen 102a bis 102h verbunden, so dass die Fördervorrichtung 111 im Sprühbetrieb Flüssigkeit 103 aus dem Flüssigkeitstank 110 saugt und zu den Zerstäuberdüsen 102a bis 102h fördert. Besonders bevorzugt ist jede Zerstäuberdüse mit einer eigenen Förderleitung über die Fördereinrichtung mit dem Flüssigkeitstank verbunden. Dabei sind die Förderleitungen insbesondere einstückig als extrudierte Kunststoffschläuche ausgebildet, wie sie aus Schlauchpumpen bekannt sind.

Die Fördervorrichtung 111 ist als Pumpe 114 nämlich Verdrängerpumpe in der Bauart einer Saugpumpe 115 ausgebildet. Die Saugpumpe 115 ist als Schlauchpumpe 116 ausgebildet und so ausgeführt, dass ein zwischen der ersten Leitung 112 und der zweiten Leitung 113 angeordneten Förderschlauch 117 im Pumpbetrieb von an einem Rotor 116a angeordneten Rollen 116b, 116c abwechselnd wälzend deformiert und hierbei vollständig abdrückt wird, wie dies in der Figur 1 im Bereich der Rolle 116b gezeigt ist.

Der Zerstäuber 101 umfasst eine Umschaltvorrichtung 118, mittels welcher eine Drehrichtung des Rotors 116a der Schlauchpumpe 116 derart umschaltbar ist, dass die Schlauchpumpe zwischen einem Förderbetrieb, bei welchen sich der Rotor 116a in eine Drehrichtung w dreht und einem Rückförderbetrieb, bei welchem sich der Rotor 116a in eine entgegen gesetzte Drehrichtung w' dreht, wechselt. Durch einen kurzzeitigen Rückförderbetrieb ist es möglich, ein ungewünschtes Austreten von Flüssigkeit 103 nach dem Sprühen zu vermeiden.

Hierbei ist es in einer Ausgestaltung vorgesehen, eine Rückförderung der Flüssigkeit 103 in den Flüssigkeitstank 110 zu vermeiden. Eine Vermeidung von Rückförderungen ist zweckmäßig, wenn die Flüssigkeit im Tank starken Hygienevorschriften unterliegt, und eine Kontamination durch bereits entnommene Flüssigkeit vermieden werden soll. Dies wird dadurch erreicht, dass die erste Leitung 112, welche wie der Förderschlauch 117 elastisch dehnbar ausgebildet ist, durch das Rückfördern zur Vergrößerung ihres Innenvolumens leicht gedehnt wird und der Flüssigkeitstank 110 durch ein nicht dargestelltes Rückschlagventil verschlossen bleibt. Um für eine Rückförderung ein großes Speichervolumen der ersten Leitung sicherzustellen, ist es vorgesehen, die erste Leitung wenigstens 1,5 und insbesondere zweimal so lange auszubilden wie dies zur Verbindung des Flüssigkeitstanks mit der Fördervorrichtung erforderlich ist. Alternativ ist es auch vorgesehen, kleine Mengen von Flüssigkeit beim Rückförderbetrieb über eine Weiche bzw. ein entsprechendes Ventil in einen Entsorgungstank zu leiten. Hierdurch ist es möglich, die zweite Leitung 113 weitgehend vollständig zu entleeren.

Alternativ ist es auch vorgesehen, Rückförderungen in den Flüssigkeitstank durch ein geöffnetes Ventil zuzulassen. Dies ist insbesondere dann von Vorteil, wenn die entnommene Flüssigkeit, beispielsweise ein Sonnenschutzmittel, aus dem Kreislauf des Zerstäubers in den Flüssigkeitstank zurückgegeben werden soll, um den Zerstäuber möglichst vollständig zu entleeren, ohne Material zu verschwenden.

Die Fördervorrichtung 101 und die Hochspannungsquelle 109 sind über das Aktivierungsmittel 106, welches durch den elektrischen Taster 107 gebildet ist, aktivierbar. Hierbei ist ein Betätigungselement 107a des Tasters 107 elektrisch leitfähig und als Gegenpol zu den Zerstäuberdüsen 102a bis 102b ausgebildet ist. Nachdem der Taster 107 zum Sprühen dauerhaft gedrückt sein muss, ist auf diese Weise eine gute Verbindung des Anwenders mit dem Bezugspotential der Sprühvorrichtung 101 sicher gestellt und eine elektrostatische Aufladung des Anwenders wird vermieden.

Der Zerstäuber 101 umfasst einen akustischen Signalgeber 119, welcher an die Kontrollelektronik 108 angeschlossen ist. Hierdurch kann der Anwender akustisch über eine korrekte oder fehlerhafte Anwendung des Zerstäubers 101 informiert werden und die Handhabung der Zerstäubers 101 damit trainiert und optimiert werden. Zusätzlich oder alternativ ist auch ein haptischer, insbesondere vibrierender Signalgeber vorgesehen, wobei dieser mit einer bestimmten Frequenz immer dann aktiv ist, wenn der Zerstäuber sprüht. Durch ein Vibrieren mit einer abweichenden Frequenz können dem Anwender weitere Informationen, z.B. eine Fehlbedienung vermittelt werden.

Der Zerstäuber 101 umfasst ein Anschlussmittel 120, wobei der Flüssigkeitstank 110 lösbar und über ein selbst schließendes Ventil 121, mit dem Anschlussmittel 120 verbunden ist. Hierdurch ist ein Kuppeln und Entkuppeln des Flüssigkeitstanks 110 mit einfachen Handbewegungen möglich und hierdurch ist auch einem ungewünschten Auslaufen des Flüssigkeitstanks 110 wirksam vorgebeugt.

Der Flüssigkeitstank 110 ist als selbst kollabierender Tank ausgebildet ist und umfasst hierzu einen Folienbeutel 110a. Alternativ kann der Flüssigkeitstank auch mit einem passiven Folgekolben ausgestattet sein. Derartige Flüssigkeitstanks lassen sich kostengünstig herstellen. Weiterhin ist der Füllstand derartiger Flüssigkeitstanks durch eine Sichtprüfung bei entsprechender Verwendung von transparenten Bauteilen und/oder entsprechender Positionierung von Öffnungen leicht feststellbar.

Die erste Leitung 112, die zweite Leitung 113 und der Förderschlauch 117 sind einteilig ausgebildet und durch einen bevorzugt einstückig ausgebildeten Verbindungsschlauch 122 gebildet. Hierdurch ist die Zahl der Verbindungsstellen reduziert, so dass die Gefahr eine Ausbildung von Ablagerungen reduziert ist.

Zwischen dem Flüssigkeitstank 110 und der Kontrollelektronik 108 sind Mittel 123 zur Identifizierung des Flüssigkeitstanks 110 umfasst, wobei diese Mittel 123 als Erfassungsmittel ausgebildet sind, welche auf dem Flüssigkeitstank aufgedruckte bzw. an dem Flüssigkeitstank gespeicherte Informationen erfassen und der Kontrollelektronik entsprechende Informationen weiterleiten. Hierdurch ist die Kontrollelektronik 108 in der Lage verschiedene Parameter wie Höhe der Hochspannung, Leistung der Pumpe usw. auf die zu versprühende Flüssigkeit 103 anzupassen. Besonders bevorzugt können hier RFID Markierungen vorgesehen werden, welche insbesondere lesbar und beschreibbar ausgebildet sind, damit auch aktuelle Informationen, wie z.B. ein Datum der ersten Benutzung des Flüssigkeitstanks, gespeichert werden können.

Der Zerstäuber 101 umfasst in der dargestellten Ausführungsform 8 Zerstäuberdüsen 102a bis 102h, welche ringartig angeordnet sind (siehe Figur 3). Hierdurch lassen sich Körperflächen von einigen Quadratzentimetern besprühen, so dass ein rascher Sprühfortschritt gewährleistet ist.

Die Zerstäuberdüsen 102a bis 102h bilden ein Düsenfeld 124 (siehe Figur 3), wobei das Düsenfeld 124 als Gitter 125 mit sich an Gitterpunkten 126a bis 126i kreuzenden Gitterlinien 127a bis 127f ausgebildet ist, wobei es insbesondere vorgesehen ist, dass die Zerstäuberdüsen 102a bis 102h Gitterpunkte paralleler Gitterlinien bilden. Durch eine derartige regelmäßige geometrische Anordnung lässt sich ein gleichmäßiger Auftrage der Flüssigkeit erreichen.

Aus den Figuren 1 und 2 ist wieder ersichtlich, dass das Gehäuse 101 einen Griffabschnitt 128, einen Kopfabschnitt 129 und einen zwischen dem Griffabschnitt 128 und dem Kopfabschnitt 129 angeordneten Mittelabschnitt 130 umfasst, wobei die Zerstäuberdüsen 102a bis 102h in dem Kopfabschnitt 129 angeordnet sind, wobei die Fördervorrichtung 111 zwischen dem Flüssigkeitstank 110 und den Zerstäuberdüsen 102a bis 102h angeordnet ist.

Die Kontrollelektronik umfasst Sensormittel S102-1 und S102-2, mit welchen an der Zerstäuberdüse 102g Spannung V102 und/oder Stromstärke A102 erfasst werden. Weiterhin umfasst die Kontrollelektronik 108 Sensormitteln S109-1 und S109-2, mit welchen an der Hochspannungsquelle 109 Spannung V109 und/oder Stromstärke A109 erfasst werden.

Die Fördervorrichtung 111 und/oder die Hochspannungsquelle 109 werden in Abhängigkeit von der an der Zerstäuberdüse 102g gemessenen Spannung V102 und/oder Stromstärke A102 und/oder in Abhängigkeit von der an der Hochspannungsquelle 109 gemessenen Spannung V109 und/oder Stromstärke A109 zur Optimierung der Flüssigkeitsabgabe des Zerstäubers 101 kontrolliert. Hierdurch kann die ordnungsgemäße Funktion der Fördervorrichtung 111 und/oder die Hochspannungsquelle 109 überwacht werden. Weiterhin erlaubt eine derartige Überwachung eine Einleitung von Verbesserungsmaßnahmen, eine Ausgabe von Fehlermeldungen, sowie eine Qualitätsüberwachung.

Weiterhin ist es vorgesehen, eine Analyse der Stromstärke A102 bzw. A107 und/oder der Spannung V102 bzw. V107 an wenigstens einer der Zerstäuberdüsen 102a bis 102h und einem elektrisch leitfähigen Abschnitt des Bedienelements 107 des Aktivierungsmittels 106 durchzuführen und einen Abgleich dieser Messungen mit Referenzwerten, welche in der Kontrollelektronik 108 gespeichert sind, durchzuführen. Hierdurch kann ebenfalls die ordnungsgemäße Funktion des Zerstäubers überwacht werden. Eine derartige Überwachung erlaubt ebenfalls eine Einleitung von Verbesserungsmaßnahmen, eine Ausgabe von Fehlermeldungen, sowie eine Qualitätsüberwachung. An dem Bedienelement 107 werden Spannung und/oder Stromstärke mit Sensormitteln S107-1 und S107-2 gemessen.

Es ist auch vorgesehen, dass durch eine Analyse von gemessener Stromstärken und/oder Spannungen ein Abstandswert AW zwischen wenigstens einer der Zerstäuberdüsen 102a bis 102h und einer zu besprühenden Oberfläche F ermittelt wird.

Weiterhin ist vorgesehen, dass durch eine Analyse gemessener Stromstärken und/oder der Spannungen ein Beschichtungsparameter BP, wie z.B. eine Dicke einer Schicht auf der zu besprühenden Oberfläche F ermittelt wird.

Es ist auch vorgesehen, dass durch eine Analyse gemessener Stromstärken und/oder Spannungen wenigstens einer der Zerstäuberdüsen 102a bis 102h eine Ausrichtung der wenigsten einen Zerstäuberdüse 102a bis 102h im Bezug zu der zu besprühenden Oberfläche F ermittelt wird.

Gemäß einer nicht dargestellten Ausführungsvariante ist es vorgesehen, dass der Zerstäuber zusätzlich zu dem ersten Flüssigkeitstank einen zweiten Flüssigkeitstank umfasst, dass der Zerstäuber zusätzlich zu der ersten Fördervorrichtung eine zweite Fördervorrichtung umfasst, dass zwischen den Fördervorrichtungen und der wenigstens einen Zerstäuberdüse eine Mischvorrichtung zwischengeschaltet ist, wobei ein Fördervolumen der ersten Fördervorrichtung und ein Fördervolumen der zweiten Fördervorrichtung derart kontrolliert werden, dass in der Mischvorrichtung eine Mischflüssigkeit mit einer vorgegebenen Zusammensetzung erzeugt wird. Hierdurch lassen sich unterschiedliche Flüssigkeiten vor dem Versprühen mischen. Durch einen Betrieb der Fördervorrichtungen mit unterschiedlicher Förderleistung lässt sich auch das Mischverhältnis bestimmen.

Wie aus der Figur 1 ersichtlich ist, ist es auch vorgesehen, dass der Zerstäuber 101 eine Heizvorrichtung 140 und eine Kühlvorrichtung 141 umfasst, wobei die Flüssigkeit 103 vor dem Austritt aus den Zerstäuberdüsen 102a bis 102h zur Erreichung einer vorgegebenen Temperatur und/oder Viskosität geheizt oder gekühlt wird.

Es ist vorgesehen, dass bei einer Aktivierung des Zerstäubers 101 zunächst der Hochspannungsgenerator 109 aktiviert wird und anschließend die Fördervorrichtung 111 aktiviert wird. Hierdurch kann sichergestellt werden, dass an den Zerstäuberdüsen 102a bis 102h ankommende Flüssigkeit 103 sofort zerstäubt wird und die Zerstäuberdüsen nicht tropfen.

Weiterhin ist es vorgesehen, dass bei einer Deaktivierung des Zerstäubers zunächst die Fördervorrichtung deaktiviert wird und anschließend der Hochspannungsgenerator deaktiviert wird. Hierdurch kann einem Tropfen der Zerstäuberdüsen ebenfalls vorgebeugt werden. Zusätzlich ist es vorgesehen, dass die Fördervorrichtung vor ihrer Deaktivierung von einer Vorwärtsförderung automatisch auf eine kurzzeitige Rückwärtsförderung umgeschaltet wird. Hierdurch wird die Flüssigkeit aus der zweiten Leitung 113 herausgesaugt, so dass ein Tropfen noch zuverlässiger vermieden wird und die für den Abschaltvorgang erforderliche Zeit verringert werden kann.

Im Ablauf für das Aktivieren ist es auch vorgesehen, dass bei einer Aktivierung des Zerstäubers 101 vor einer Aktivierung des Hochspannungsgenerators 109 die Zerstäuberdüsen 102a bis 102h aus der Ruhestellung R102 in die Arbeitsstellung A102 verbracht werden, wobei hierzu die Zerstäuberdüsen 102a bis 102h mittels des Hubzylinders 134 bewegt werden.

Im Ablauf für das Deaktivieren ist es auch vorgesehen, dass bei einer Deaktivierung des Zerstäubers 101 nach einer Deaktivierung des Hochspannungsgenerators 109 die Zerstäuberdüsen 102a bis 102h aus der Arbeitsstellung A102 in die Ruhestellung R102 verbracht werden, wobei hierzu die Zerstäuberdüsen 102a bis 102h wieder mittels des Hubzylinders 134 bewegt werden.

Alternativ oder ergänzend zu Bewegung der Zerstäuberdüsen kann auch ein Gehäuseteil als Schieber ausgebildet werden (nicht dargestellt), welcher zur Abdeckung im deaktivierten Zustand vor die Zerstäuberdüsen geschoben wird und dabei bevorzugt einen Ein-Aus-Schalter bedient.

### Bezugszeichenliste:

- 101: Zerstäuber (erste Variante)
- 102a - 102h: Zerstäuberdüse
- 103: Flüssigkeiten
- 104: Gehäuse
- 105: Energiequelle
- 106: Aktivierungsmittel
- 107: elektrischer Taster
- 107a: Betätigungselement von 107
- 108: Kontrollelektronik
- 109: Hochspannungsquelle
- 110: Flüssigkeitstank
- 110a: Folienbeutel
- 111: Fördervorrichtung
- 112: erste Leitung
- 113: zweite Leitung
- 114: Pumpe
- 115: Saugpumpe
- 116: Schlauchpumpe
- 117: Förderschlauch
- 116a: Rotor
- 116b, 116c: Rolle
- 117: Förderschlauch
- 118: Umschaltvorrichtung
- 119: Signalgeber
- 120: Anschlussmittel
- 121: Ventil
- 122: Verbindungsschlauch
- 123: Mittel zur Identifizierung des Flüssigkeitstanks
- 124: Düsenfeld
- 125: Gitter
- 126a - 126i: Gitterpunkt
- 127a - 127f: Gitterlinie
- 128: Griffabschnitt
- 129: Kopfabschnitt
- 130: Mittelabschnitt
- 131: Innenraum
- 132: Außenseite
- 133: Basisplatte
- 134: Hubzylinder

- 140: Heizvorrichtung
- 141: Kühlvorrichtung

- AW: Abstandswert
- BP: Beschichtungsparameter
- F: zu beschichtende Fläche

- S102-1, S102-2: Sensormittel an 102g
- S107-1, S107-2: Sensormittel an 107
- S109-1, S109-2: Sensormittel an 109

## Patentansprüche

1. Verfahren zur Steuerung eines elektrostatischen Zerstäubers (101) für Flüssigkeiten (103), wobei der Zerstäuber (101) einen Flüssigkeitstank (110) und eine Fördervorrichtung (111) für Flüssigkeit (103) aus dem Flüssigkeitstank (110), eine Hochspannungsquelle (109) sowie Zerstäuberdüsen (102a - 102h) für die Zerstäubung von Flüssigkeit (103) umfasst, wobei die Zerstäuberdüsen (102a - 102h) an der Hochspannungsquelle (109) angeschlossen sind, wobei mittels Sensormitteln (S102-1, S102-2) einer Kontrollelektronik (108) an wenigstens einer der Zerstäuberdüsen (102a - 102h) Spannung und/oder Stromstärke erfasst werden und/oder dass mittels Sensormitteln (S109-1, S109-2) der Kontrollelektronik (108) Spannung und/oder Stromstärke an der Hochspannungsquelle (109) erfasst werden, **dadurch gekennzeichnet, dass** bei einer Deaktivierung des Zerstäubers (101) zunächst die Fördervorrichtung (111) deaktiviert wird und anschließend der Hochspannungsgenerator (109) deaktiviert wird.

2. Verfahren nach Anspruch 1, **dadurch gekennzeichnet, dass** der Zerstäuber (101)
- ein Gehäuse (104),
- eine elektrische Energiequelle (105), durch welche die Hochspannungsquelle (109) versorgt wird,
- ein Aktivierungsmittel (106), durch welches der Zerstäuber (101) aktiviert wird, und
- die Kontrollelektronik (108), durch welche die Flüssigkeitsabgabe des Zerstäubers (101) kontrolliert wird, umfasst.

3. Verfahren nach einem der vorangegangenen Ansprüche, **dadurch gekennzeichnet,**
- **dass** die Fördervorrichtung (111) zwischen dem Flüssigkeitstank (110) und den Zerstäuberdüsen (102a - 102h) angeordnet ist,
- wobei die Fördervorrichtung (111) durch eine erste Leitung (112) mit dem Flüssigkeitstank (110) verbunden ist und von der Fördervorrichtung (111) aus dem Flüssigkeitstank (110) Flüssigkeit (103) angesaugt wird und
- wobei die Fördervorrichtung (111) durch eine zweite Leitung (113) mit den Zerstäuberdüsen (102a - 102h) verbunden ist und von der Fördervorrichtung (111) Flüssigkeit (103) zu den Zerstäuberdüsen (102a - 102h) gefördert wird
- wobei bevorzugt die erste Leitung und die zweite Leitung als einstückig extrudierter Schlauch ausgebildet ist.

4. Verfahren nach einem der vorangegangenen Ansprüche, **dadurch gekennzeichnet, dass** die Fördervorrichtung (111) und/oder die Hochspannungsquelle (109) in Abhängigkeit von der an der wenigstens einen Zerstäuberdüse (102a - 102h) gemessenen Spannung und/oder Stromstärke und/oder in Abhängigkeit von der an der Hochspannungsquelle (109) gemessenen Spannung und/oder Stromstärke zur Optimierung der Flüssigkeitsabgabe des Zerstäubers (101) kontrolliert, insbesondere geregelt wird.

5. Verfahren nach einem der vorangegangenen Ansprüche, **dadurch gekennzeichnet, dass** eine Analyse der Stromstärke und/oder der Spannung an der wenigstens einen Zerstäuberdüse (102a - 102h) und einem elektrisch leitfähigen Abschnitt des Gehäuses (103) oder eines Bedienelements (107a) eines Aktivierungsmittels (106) erfolgen und ein Abgleich dieser Messungen mit Referenzwerten durchgeführt wird.

6. Verfahren nach einem der vorangegangenen Ansprüche, **dadurch gekennzeichnet, dass** durch eine Analyse von gemessenen Stromstärken und/oder Spannungen ein Abstandswert (AW) zwischen der wenigstens einen Zerstäuberdüse (102a - 102h) und einer zu besprühenden Oberfläche (F) ermittelt wird.

7. Verfahren nach einem der vorangegangenen Ansprüche, **dadurch gekennzeichnet, dass** durch eine Analyse von gemessenen Stromstärken und/oder Spannungen ein Beschichtungsparameter (BP) einer zu besprühenden Oberfläche (F) ermittelt wird.

8. Verfahren nach einem der vorangegangenen Ansprüche, **dadurch gekennzeichnet, dass** durch eine Analyse von gemessenen Stromstärken und/oder Spannungen der wenigstens einen Zerstäuberdüse (102a - 102h) eine Ausrichtung der wenigsten einen Zerstäuberdüse (102a - 102h) im Bezug zu einer zu besprühenden Oberfläche (F) ermittelt wird.

9. Verfahren nach einem der vorangegangenen Ansprüche, **dadurch gekennzeichnet, dass** der Zerstäuber (101) zusätzlich zu dem ersten Flüssigkeitstank (110) einen zweiten Flüssigkeitstank umfasst, dass der Zerstäuber (101) zusätzlich zu der ersten Fördervorrichtung (111) eine zweite Fördervorrichtung umfasst, dass zwischen den Fördervorrichtungen und der wenigstens einen Zerstäuberdüse (102a - 102h) eine Mischvorrichtung zwischengeschaltet ist, wobei ein Fördervolumen der ersten Fördervorrichtung und ein Fördervolumen der zweiten Fördervorrichtung derart kontrolliert werden, dass in der Mischvorrichtung eine Mischflüssigkeit mit einer vorgegebenen Zusammensetzung erzeugt wird.

10. Verfahren nach einem der vorangegangenen Ansprüche, **dadurch gekennzeichnet, dass** der Zerstäuber (101) eine Heizvorrichtung (140) und/oder eine Kühlvorrichtung (141) umfasst, wobei die Flüssigkeit (103) vor dem Austritt aus den Zerstäuberdüsen (102a - 102h) zur Erreichung einer vorgegebenen Temperatur und/oder Viskosität geheizt oder gekühlt wird.

11. Verfahren nach einem der vorangegangenen Ansprüche, **dadurch gekennzeichnet, dass** bei einer Aktivierung des Zerstäubers (101) zunächst der Hochspannungsgenerator (109) aktiviert wird und anschließend die Fördervorrichtung (111) aktiviert wird.

12. Verfahren nach einem der vorangegangenen Ansprüche, **dadurch gekennzeichnet, dass** die Fördervorrichtung (111) vor ihrer Deaktivierung von einer Vorwärtsförderung automatisch auf eine kurzzeitige Rückwärtsförderung umgeschaltet wird.

13. Verfahren nach Anspruch 11 oder 12, **dadurch gekennzeichnet, dass** bei einer Aktivierung des Zerstäubers (101) vor einer Aktivierung des Hochspannungsgenerators (109) die Zerstäuberdüsen (102a - 102h) aus einer Ruhestellung (R101) in eine Arbeitsstellung (A101) verbracht werden, wobei hierzu die Zerstäuberdüsen (102a - 102h) und/oder das Gehäuse (104) bewegt wird und/oder wobei wenigstens ein Gehäuseabschnitt, insbesondere ein Schieber relativ zu den Zerstäuberdüsen bewegt wird.

14. Verfahren nach Anspruch 11 oder 12, **dadurch gekennzeichnet, dass** bei einer Deaktivierung des Zerstäubers (101) nach einer Deaktivierung des Hochspannungsgenerators (109) die Zerstäuberdüsen (102a - 102h) aus einer Arbeitsstellung (A101) in eine Ruhestellung (R101) verbracht werden, wobei hierzu die Zerstäuberdüsen (102a - 102h) und/oder das Gehäuse (104), zumindest ein Gehäuseabschnitt bewegt wird.

## Claims

1. Method for controlling an electrostatic atomizer (101) for liquids (103), the atomizer (101) comprising a liquid tank (110) and a delivery device (111) for liquid (103) from the liquid tank (110), a high-voltage source (109) and also atomizer nozzles (102a - 102h) for the atomization of liquid (103), the atomizer nozzles (102a - 102h) being connected to the high-voltage source (109), the voltage and/or the current intensity at at least one of the atomizer nozzles (102a - 102h) being detected by means of sensor means (S102-1, S102-2) of control electronics (108) and/or the voltage and/or the current intensity at the high-voltage source (109) being detected by means of sensor means (S109-1, S109-2) of the control electronics (108), **characterized in that**, when there is a deactivation of the atomizer (101), first the delivery device (111) is deactivated and then the high-voltage generator (109) is deactivated.

2. Method according to Claim 1, **characterized in that** the atomizer (101) comprises
- a housing (104),
- an electrical energy source (105), by which the high-voltage source (109) is supplied,
- an activation means (106), by which the atomizer (101) is activated, and
- the control electronics (108), by which the dispensing of liquid by the atomizer (101) is controlled.

3. Method according to one of the preceding claims, **characterized**
- **in that** the delivery device (111) is arranged between the liquid tank (110) and the atomizer nozzles (102a - 102h),
- the delivery device (111) being connected to the liquid tank (110) by a first line (112) and liquid (103) being sucked out of the liquid tank (110) by the delivery device (111) and
- the delivery device (111) being connected to the atomizer nozzles (102a - 102h) by a second line (113) and liquid (103) being delivered by the delivery device (111) to the atomizer nozzles (102a - 102h),
- the first line and the second line preferably being formed as a hose extruded in one piece.

4. Method according to one of the preceding claims, **characterized in that** the delivery device (111) and/or the high-voltage source (109) are controlled, in particular in a closed-loop manner, in dependence on the voltage and/or the current intensity measured at the at least one atomizer nozzle (102a - 102h) and/or in dependence on the voltage and/or the current intensity measured at the high-voltage source (109) to optimize the dispensing of liquid by the atomizer (101).

5. Method according to one of the preceding claims, **characterized in that** an analysis of the current intensity and/or the voltage at the at least one atomizer nozzle (102a - 102h) and an electrically conductive portion of the housing (103) or of an operating element (107a) of an activation means (106) is performed and a comparison of these measurements with reference values is carried out.

6. Method according to one of the preceding claims, **characterized in that**, by an analysis of measured current intensities and/or voltages, a distance value (AW) between the at least one atomizer nozzle (102a - 102h) and a surface (F) to be sprayed is determined.

7. Method according to one of the preceding claims, **characterized in that**, by an analysis of measured current intensities and/or voltages, a coating parameter (BP) of a surface (F) to be sprayed is determined.

8. Method according to one of the preceding claims, **characterized in that**, by an analysis of measured current intensities and/or voltages of the at least one atomizer nozzle (102a - 102h), an alignment of the at least one atomizer nozzle (102a - 102h) with respect to a surface (F) to be sprayed is determined.

9. Method according to one of the preceding claims, **characterized in that** the atomizer (101) comprises in addition to the first liquid tank (110) a second liquid tank, **in that** the atomizer (101) comprises in addition to the first delivery device (111) a second delivery device, **in that** arranged between the delivery devices and the at least one atomizer nozzle (102a - 102h) is a mixing device, a delivery volume of the first delivery device and a delivery volume of the second delivery device being controlled in such a way that a mixed liquid of a prescribed composition is produced in the mixing device.

10. Method according to one of the preceding claims, **characterized in that** the atomizer (101) comprises a heating device (140) and/or a cooling device (141), the liquid (103) being heated or cooled to achieve a prescribed temperature and/or viscosity before leaving the atomizer nozzles (102a - 102h).

11. Method according to one of the preceding claims, **characterized in that**, when there is an activation of the atomizer (101), first the high-voltage generator (109) is activated and then the delivery device (111) is activated.

12. Method according to one of the preceding claims, **characterized in that**, before its deactivation, the delivery device (111) is automatically switched from forward delivery to brief backward delivery.

13. Method according to Claim 11 or 12, **characterized in that**, when there is an activation of the atomizer (101) before an activation of the high-voltage generator (109), the atomizer nozzles (102a - 102h) are brought from an inactive position (R101) into an active position (A101), the atomizer nozzles (102a - 102h) and/or the housing (104) being moved for this purpose and/or at least one housing portion, in particular a slide, being moved in relation to the atomizer nozzles.

14. Method according to Claim 11 or 12, **characterized in that**, when there is a deactivation of the atomizer (101) after a deactivation of the high-voltage generator (109), the atomizer nozzles (102a - 102h) are brought from an active position (A101) into an inactive position (R101), the atomizer nozzles (102a - 102h) and/or the housing (104), at least one housing portion, being moved for this purpose.

## Revendications

1. Procédé de commande d'un pulvérisateur électrostatique (101) destiné à des liquides (103), le pulvérisateur (101) comprenant un réservoir de liquide (110) et un dispositif (111) de transport du liquide (103) depuis le réservoir de liquide (110), une source à haute tension (109) et des buses de pulvérisateur (102a-102h) destinées à la pulvérisation du liquide (103), les buses de pulvérisateur (102a-102h) étant raccordées à la source à haute tension (109), des moyens de détection (S102-1, S102-2) d'une électronique de commande (108) permettant de détecter la tension et/ou l'intensité de courant au niveau d'au moins une des buses de pulvérisateur (102a-102h) et/ou des moyens de détection (S109-1, S109-2) de l'électronique de commande (108) permettant de détecter la tension et/ou l'intensité de courant au niveau de la source à haute tension (109), **caractérisé en ce que**, lorsque le pulvérisateur (101) est désactivé, le dispositif de transport (111) est d'abord désactivé puis le générateur de haute tension (109) est désactivé.

2. Procédé selon la revendication 1, **caractérisé en ce que** le pulvérisateur (101) comprend
- un boîtier (104),
- une source d'énergie électrique (105) permettant d'alimenter la source à haute tension (109),
- un moyen d'activation (106) permettant d'activer le pulvérisateur (101), et
- l'électronique de commande (108) permettant de commander la distribution de liquide du pulvérisateur (101) .

3. Procédé selon l'une des revendications précédentes, **caractérisé en ce que**
- le dispositif de transport (111) est disposé entre le réservoir de liquide (110) et les buses de pulvérisation (102a-102h),
- le dispositif de transport (111) étant relié au réservoir de liquide (110) par une première conduite (112) et le liquide (103) étant aspiré du réservoir de liquide (110) par le dispositif de transport (111) et
- le dispositif de transport (111) étant relié aux buses de pulvérisation (102a-102h) par une deuxième conduite (113) et le liquide (103) étant transporté du dispositif de transport (111) aux buses de pulvérisation (102a-102h),
- la première conduite et la deuxième conduite étant de préférence conçues comme un tuyau extrudé d'une seule pièce.

4. Procédé selon l'une des revendications précédentes, **caractérisé en ce que** le dispositif de transport (111) et/ou la source à haute tension (109) est commandée, notamment régulée, en fonction de la tension et/ou de l'intensité de courant mesurée au niveau de l'au moins une buse de pulvérisateur (102a-102h) et/ou en fonction de la tension et/ou de l'intensité de courant mesurée au niveau de la source à haute tension (109) afin d'optimiser la distribution de liquide du pulvérisateur (101) .

5. Procédé selon l'une des revendications précédentes, **caractérisé en ce qu'**une analyse de l'intensité de courant et/ou de la tension au niveau de l'au moins une buse de pulvérisateur (102a-102h) et d'une portion électriquement conductrice du boîtier (103) ou d'un élément de commande (107a) d'un moyen d'activation (106) est effectuée et ces mesures sont comparées à des valeurs de référence.

6. Procédé selon l'une des revendications précédentes, **caractérisé en ce qu'**une valeur de distance (AW) entre l'au moins une buse de pulvérisateur (102a-102h) et une surface (F) à pulvériser est déterminée par une analyse des intensités de courant et/ou des tensions mesurées.

7. Procédé selon l'une des revendications précédentes, **caractérisé en ce qu'**un paramètre de revêtement (BP) d'une surface (F) à pulvériser est déterminé par une analyse des intensités de courant et/ou des tensions mesurées.

8. Procédé selon l'une des revendications précédentes, **caractérisé en ce qu'**une orientation de l'au moins une buse de pulvérisateur (102a-102h) par rapport à une surface (F) à pulvériser est déterminée par une analyse des intensités de courant et/ou des tensions mesurées de l'au moins une buse de pulvérisateur (102a-102h).

9. Procédé selon l'une des revendications précédentes, **caractérisé en ce que** le pulvérisateur (101) comprend un deuxième réservoir de liquide en plus du premier réservoir de liquide (110), **en ce que** le pulvérisateur (101) comprend un deuxième dispositif de transport en plus du premier dispositif de transport (111), **en ce qu'**un dispositif de mélange est interposé entre les dispositifs de transport et l'au moins une buse de pulvérisateur (102a-102h), un volume de transport du premier dispositif de transport et un volume de transport du deuxième dispositif de transport étant commandés de manière à générer dans le dispositif de mélange un liquide de mélange de composition spécifiée.

10. Procédé selon l'une des revendications précédentes, **caractérisé en ce que** le pulvérisateur (101) comprend un dispositif de chauffage (140) et/ou un dispositif de refroidissement (141), le liquide (103) étant chauffé ou refroidi avant de sortir des buses de pulvérisateur (102a-102h) afin d'atteindre une température et/ou une viscosité spécifiées.

11. Procédé selon l'une des revendications précédentes, **caractérisé en ce que**, lorsque le pulvérisateur (101) est activé, le générateur de haute tension (109) est d'abord activé et le dispositif de transport (111) est ensuite activé.

12. Procédé selon l'une des revendications précédentes, **caractérisé en ce que** le dispositif de transport (111) est commuté automatiquement d'un transport en avant à un transport en arrière de courte durée avant sa désactivation.

13. Procédé selon la revendication 11 ou 12, **caractérisé en ce que**, lorsque le pulvérisateur (101) est activé avant que le générateur de haute tension (109) ne soit activé, les buses de pulvérisateur (102a-102h) sont déplacées d'une position de repos (R101) à une position de travail (A101), les buses de pulvérisateur (102a-102h) et/ou le boîtier (104) étant pour cela déplacés et/ou au moins une portion de boîtier, en particulier un coulisseau, étant déplacé(e) par rapport aux buses de pulvérisateur.

14. Procédé selon la revendication 11 ou 12, **caractérisé en ce que**, lorsque le pulvérisateur (101) est désactivé après que le générateur de haute tension (109) a été désactivé, les buses de pulvérisateur (102a-102h) sont déplacées d'une position de travail (A101) à une position de repos (R101), les buses de pulvérisateur (102a-102h) et/ou le boîtier (104), au moins une portion de boîtier, étant pour cela déplacés.
